(19) Europäisches Patentamt
European Patent Office
Office européen des brevets

(11) Veröffentlichungsnummer : **0 027 940**
**B2**

(12) # NEUE EUROPÄISCHE PATENTSCHRIFT

(45) Veröffentlichungstag der neuen Patentschrift : **30.12.86**

(21) Anmeldenummer : **80106196.1**

(22) Anmeldetag : **11.10.80**

(51) Int. Cl.⁴ : **C 07 C125/065**, C 07 C125/073, C 07 D213/75, C 07 D295/22, C 07 D307/82, C 07 D333/48

(54) **Verfahren zur Herstellung von Urethanen.**

(30) Priorität : **27.10.79 DE 2943481**

(43) Veröffentlichungstag der Anmeldung : **06.05.81 Patentblatt 81/18**

(45) Bekanntmachung des Hinweises auf die Patenterteilung : **14.09.83 Patentblatt 83/37**

(45) Bekanntmachung des Hinweises auf die Entscheidung über den Einspruch : **30.12.86 Patentblatt 86/52**

(84) Benannte Vertragsstaaten :
**AT BE CH DE FR GB IT LI NL SE**

(56) Entgegenhaltungen :
**EP-A- 0 018 583**
**EP-A- 0 018 586**
**EP-A- 0 019 109**
**US-A- 2 409 712**
**US-A- 2 806 051**

(73) Patentinhaber : **BAYER AG**
**Konzernverwaltung RP Patentabteilung**
**D-5090 Leverkusen 1 Bayerwerk (DE)**

(72) Erfinder : **König, Klaus, Dr.**
**Heymannstrasse 50**
**D-5090 Leverkusen 1 (DE)**
Erfinder : **Findeisen, Kurt, Dr.**
**In der Follmühle 10**
**D-5068 Odenthal 2 (DE)**
Erfinder : **Sundermann, Rudolf, Dr.**
**Treptower Strasse 5**
**D-5090 Leverkusen (DE)**
Erfinder : **Penninger, Stefan, Dr.**
**Pommernallee 5**
**D-4047 Dormagen (DE)**
Erfinder : **Fauss, Rudolf, Dr.**
**Gerstenkamp 10**
**D-5000 Köln 80 (DE)**

**EP 0 027 940 B2**

**Beschreibung**

Die vorliegende Erfindung betrifft ein verbessertes Verfahren zur Herstellung von Urethanen durch Umsetzung von Harnstoff oder Biuret mit Aminen und Alkoholen unter Verwendung bestimmter, nachstehend näher beschriebener Katalysatoren für die Umsetzung.

Urethane entstehen bekanntlich u. a. durch Umsetzung von organischen Isocyanaten mit Alkoholen. Diese Umsetzung ist reversibel, d. h. Urethane können thermisch in das ihnen zugrundeliegende Isocyanat und den ihnen zugrundeliegenden Alkohol gespalten werden (vgl. z. B. US-PS 24 09 712). Thermisch in Isocyanate spaltbare Urethane sind daher interessante Ausgangsmaterialien zur Herstellung dieser Isocyanate, die bislang weltweit in größtem Umfang durch Umsetzung von primären Aminen mit Phosgen hergestellt werden. Die phosgenfreie Herstellung von Urethanen und deren anschließende thermische Spaltung stellt jedoch eine interessante Alternative zu der genannten großtechnischen Isocyanatherstellung dar. Eine Methode zur phosgenfreien Herstellung von Urethanen besteht in der Umsetzung von Harnstoff mit Aminen und Alkohol (vgl. z. B. US-PS 24 09 712 oder US-PS 28 06 051). Diese Methode des Standes der Technik ist jedoch mit dem Nachteil behaftet, daß die Urethane nur in unzureichender Ausbeute und in stark mit Nebenprodukten verunreinigter Form erhalten werden. Eines der wesentlichen Nebenprodukte ist beispielsweise das Umsetzungsprodukt aus Alkohol und Harnstoff, ein N-unsubstituierter Carbaminsäureester :

$$R{-}OH + H_2N{-}CO{-}NH_2 \longrightarrow R{-}O{-}\overset{\overset{\Large O}{\|}}{C}{-}NH_2 + NH_3$$

Es war daher die Aufgabe der vorliegenden Erfindung, das bekannte Verfahren zur Herstellung von Urethanen durch Umsetzung von Harnstoff mit Alkoholen und Aminen so zu verbessern, daß die Bildung von unerwünschten Nebenprodukten weitgehend vermieden wird und die Urethane in verbesserter Ausbeute und in von unerwünschten Nebenprodukten freier Form erhalten werden.

Überraschenderweise wurde nunmehr gefunden, daß diese Aufgabe durch dit Mitverwendung von bestimmten, nachstehend näher beschriebenen Katalysatoren gelöst werden kann. Gleichzeitig wurde gefunden, daß bei der Umsetzung anstelle von Harnstoff auch dessen höhere Homologe, d. h. Polyurete, eingesetzt werden können.

Gegenstand der vorliegenden Erfindung ist somit ein Verfahren zur Herstellung von Urethanen durch Umsetzung von a) Harnstoff oder Polyureten mit b) primären Aminen und c) Alkoholen im Temperaturbereich von 120° bis 350 °C in Gegenwart von Katalysatoren, wobei im Falle der Herstellung von Aryl-mono-, -di- und/oder -polyurethanen die zwingende Anwesenheit von O-Alkylcarbamidsäureestern im Ausgangsgemisch, und zwar in einer solchen Menge, daß das Verhältnis von Aminogruppen der primären aromatischen Amine zu O-Alkylcarbamidsäureestern bei 1 : 0,5 oder darunter liegt, ausgenommen ist, dadurch gekennzeichnet, daß man die Umsetzung in Gegenwart von beliebigen Veresterungskatalysatoren für Carbonsäuren durchführt mit der Einschränkung, daß im Falle der Herstellung von Di- und/oder Polyurethanen die Veresterungskatalysatoren aus der Gruppe bestehend aus (i) unter den Reaktionsbedingungen inerten anorganischen oder organischen Basen, (ii) Lewis Säuren, (iii) Salzen bzw. Komplexverbindungen von Übergangsmetallen mit Ausnahme der Alkoholate des Titans, und (iv) Bleinaphthenat, Acetylacetonaten von Blei, Aluminium oder Magnesium, Dibutylzinndilaurat, Dibutylzinndiacetat, Di-(2-ethylhexyl)-zinnoxid, Dioctylzinnoxid, Zinnsalzen von $C_1$-$C_{20}$-Carbonsäuren, sowie Acetaten, Chloriden, Sulfaten oder Octoaten des zweiwertigen Bleis ausgewählt werden.

In den veröffentlichten, prioritätsälteren, jedoch einen nach dem 27.10.1979 liegenden europäischen Anmeldetag aufweisenden europäischen Patentanmeldungen 80 102 170.0 (0 019 109) bzw. 80 102 182.5 (0 018 586) werden ebenfalls Verfahren zur Herstellung von aromatischen bzw. aliphatischen und/oder cycloaliphatischen Di- und/oder Polyurethanen aus den obengenannten Ausgangsmaterialien unter Mitverwendung von Katalysatoren beschrieben. In den diesen europäischen Patentanmeldungen zugrundeliegenden Prioritätsunterlagen werden jedoch lediglich Alkoholate bestimmter Metalle als Katalysatoren empfohlen. Alkoholate stellen jedoch keine Verbindungen der oben unter (i)-(iii) genannten Art, insbesondere keine unter den Raktionsbedingungen inerte anorganische oder organische Basen dar, da die Alkoholkomponente der Alkoholate zumindest teilweise an der Reaktion teilnehmen würde (Gleichgewichtseinstellung zwischen dem als Ausgangsmaterial eingesetzten Alkohol und dem in Form des Alkoholats eingeführten Alkohol).

Die Herstellung von Monourethanen wird in den genannten Veröffentlichungen nicht angesprochen.

In der veröffentlichten, prioritätsälteren, europäischen Patentanmeldung 80 102 179.1 (00 18583) wird ein Verfahren zur Herstellung von Aryl-mono-, -di- und/oder -polyurethanen aus primären aromatischen Mono-, Di- und/oder Polyaminen und O-Alkylcarbamidsäureestern in Gegenwart von Katalysatoren beschrieben, wobei das Verhältnis von Aminogruppen der primären aromatischen Amine zu O-Alkylcarbamidsäureestern bei 1 : 0,5 oder darunter liegt. Ein derartiges Verfahren ist nicht Gegenstand der vorliegenden Erfindung.

Das erfindungsgemäße Verfahren eignet sich insbesondere zur Herstellung von Urethanen der

allgemeinen Formel

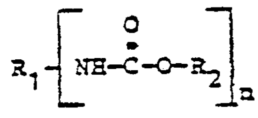

in welcher

$R_1$ für einen gegebenenfalls substituierten aliphatischen Kohlenwasserstoffrest mit 1-18 Kohlenstoffatomen, einen gegebenenfalls substituierten cycloaliphatischen Kohlenwasserstoffrest mit 3 bis 18 Kohlenstoffatomen, einen gegebenenfalls substituierten aromatischen Kohlenwasserstoffrest mit 6 bis 15 Kohlenstoffatomen, einen gegebenenfalls substituierten araliphatischen Kohlenwasserstoffrest mit 7 bis 14 Kohlenstoffatomen, oder für einen gegebenenfalls substituierten 5- oder 6-gliedrigen heterocyclischen Rest steht, der zusätzlich noch mit einem Benzolring anelliert sein kann,

$R_2$ für einen gegebenenfalls substituierten Alkylrest mit 1 bis 20 Kohlenstoffatomen, einen gegebenenfalls substituierten. Cycloalkylrest mit 3 bis 16 Kohlenstoffatomen und einen gegebenenfalls substituierten Aralkylrest mit 7 bis 14 Kohlenstoffatomen und

n eine ganze Zahl von 1 bis 3 bedeuten,

wobei im Falle von n = 2 oder 3 zwischen 2 mit dem Rest $R_1$ verknüpften Urethangruppen mindestens 2 Kohlenstoffatome angeordnet sind.

Als Substituenten für die aliphatischen oder cycloaliphatischen Reste $R_1$ bzw. $R_2$ kommen beispielsweise $C_6$-$C_{10}$-Aroxy-, $C_1$-$C_6$-Alkoxy-, $C_1$-$C_6$-Alkoxy-$C_2$-$C_4$-alkoxy-, $C_1$-$C_6$-Acyl-, $C_1$-$C_6$-Alkylmercapto-, $C_6$-$C_{10}$-Arylmercapto-$C_1$-$C_{12}$-Alkyl-carbonyl-, Bis-($C_1$-$C_8$-Alkyl)-amino-, $C_1$-$C_6$-Acylamino-, Nitro-, Cyano- oder Rhodanoreste in Betracht.

Als Substituenten für die aromatischen oder araliphatischen Reste $R_1$ bzw. $R_2$ kommen neben diesen Substituenten beispielsweise auch $C_1$-$C_{12}$-Alkyl-, $C_1$-$C_{12}$-Alkylsulfonyl-, $C_6$-$C_{10}$-Arylsulfonyl-, $C_1$-$C_{12}$-Alkylsulfonsäureester-, oder Sulfonamidreste in Betracht.

Bevorzugte erfindungsgemäße Verfahrensprodukte sind jedoch solche der genannten allgemeinen Formel, für welche

$R_1$ für einen aliphatischen Kohlenwasserstoffrest mit 3 bis 18 Kohlenstoffatomen, einen cycloaliphatischen Kohlenwasserstoffrest mit 6 bis 15 Kohlenstoffatomen oder einen gegebenenfalls Methyl-, Methoxy- oder Chlor-substituierten und gegebenenfalls Methylenbrücken aufweisenden aromatischen Kohlenwasserstoffrest mit insgesamt 6 bis 15 Kohlenstoffatomen steht.

$R_2$ für einen $C_1$-$C_4$-Alkoxy- oder $C_1$-$C_4$-Alkoxy-$C_2$-$C_4$-alkoxy-substituierten oder unsubstituierten aliphatischen Kohlenwasserstoffrest mit 1 bis 18, insbesondere 1 bis 4 Kohlenstoffatomen steht, wie er durch Entfernung der Hydroxylgruppe aus einem einerwertigen unsubstituierten primären oder sekundären aliphatischen Alkohol erhalten wird oder für einen Cyclohexyl- oder 2-Phenyl-ethyl-Rest steht und

n 1 oder 2 bedeutet.

Ausgangsmaterialien für das erfindungsgemäße Verfahren sind

a) Harnstoff oder Polyurete, wie insbesondere Biuret, Triuret oder Tetrauret der Formel

$$H_2N—(CO—NH)_m—H$$

in welcher

m für eine ganze Zahl von 1 bis 4 steht, bzw. Gemische derartiger Verbindungen.

Weitere Ausgangsmaterialien für das erfindungsgemäße Verfahren sind

b) primäre Amine der Formel

$$R_1(NH_2)n$$

in welcher

$R_1$ und n die bereits genannte Bedeutung haben.

Beispiele geeigneter Amine sind Methylamin, Ethylamin, Propylamin, Isopropylamin, Butylamin, iso-Butylamin, tert.-Butylamin, Hexylamin, Dodecylamin, 2-Ethylhexylamin, Tetradecylamin, Hexadecylamin, Octadecylamin, Allylamin, 1,4-Diaminobutan, 1,6-Diaminohexan, 2,5-Dimethyl-2,5-hexandiamin, Trimethylhexamethylendiamin, 2-Methoxyethylamin, 3-Ethoxypropylamin, 3-Butoxypropylamin, 1,4-Butandiol-bis-(3-aminopropylether), 3-Aminopropansäure-2-methylpropylester, 6-Amino-hexanitril, Lysinester, 1,1-Aminoundecansäureester, Cyclohexylamin, Cyclohexannuthanamin, Trimethylcyclohexylamin, 2-Norbornylmethylamin, Anilin, o,m,p-Chloranilin, 2,3-, 2,4-, 2,5-, 2-6-Dichloranilin, 3,4-Dichloranilin, p,o-Nitroanilin, m,o,p-Tolylamin, 3-Trifluormethylanilin, 3-Chlor-4-methylanilin, Benzylamin, Phenylcyclohexylamin, Naphthylamin, 1,4-Diaminocyclohexan, 2,4-, 2,6-Diamino-1-methylcyclohexan, 5-Amino-1-aminomethyl-1,3,3-trimethylcyclohexan, 4,4'-Diaminodicyclohexylmethan, 4,4'-Diamino-3,3'-dimethyl-dicyclohexylmethan, 1,3-Diaminobenzol, 1,4-Diaminobenzol, 2-Chlor-1,4-diaminobenzol, 2,4-Diaminotoluol, 2,6-Diaminotoluol (und Gemische mit 2,4-), 2-(N-Ethyl-amino)-4-aminotoluol, 1,3-Diamino-2-methylbenzol, 1,3-Bisaminomethylbenzol, 1,3-Bisaminomethyl-4,6-dimethylbenzol, 1,3-Diamino-2,6-(4,6)-diethyl-4-methylbenzol, 1,3-Diamino-2,4,6-triisopropylbenzol, 1,5-Diamino-naphthalin, 2,7-Diaminonaphthalin, Benzidin, 3,3'-Dichlorbenzidin, 4,4'-Diaminodiphenylmethan (und Rohwaren), 3,3'-Dichlor-4,4'-diamino-diphenylmethan, 2,2-Bis-(4-aminophenyl)-propan, 1,1'-Bis-(4-aminophenyl)-cyclohexan, 1,1'-Bis-84-amino-3-methylphenyl)-cyclohexan, 4,4',4''-Triaminotriphenylmethan, 4,4'-Diaminodiphenylether, 4,4',4''-Triaminotriphenylthiophosphat, p-Methoxyanilin, p-Ethoxyanilin, 1-(4-Chlorphenoxy)-4-aminobenzol, 2,4-Diaminodiphenylether, m-Aminobenzoesäureester, p-Aminobenzoesäureester, 3,5-Diamino-2-methyl-diphenylmethan, 3,5-Diamino-4-methyl-diphenylmethan (und Gemische), 3,5-Diamino-4-methyldicyclohexylmethan, 3,5-Diamino-2-methyl-dicyclohexylmethan (und Gemische), 3,5,4'-Triamino-4-methyl-diphenylmethan, 3,5,4'-Triamino-2-methyl-diphenylmethan, 3,5,2'-Triamino-4-methyldiphenylmethan, 3,5,2'-Triamino-2-methyl-diphenylmethan (und Gemische), 3,5,4'-Triamino-4-methyldicyclohexylmethan, 3,5,4'-Triamino-2-methyl-dicyclohexylmethan, 3,5,2'-Triamino-4-methyl-dicyclohexylmethan, 3,5,2'-Triamino-2-methyl-dicyclohexylmethan (und Gemische), Dibenzoduranamin, 1-Aziridinpropanamin, 4-Pyridinmethanamin, 2-Pyridinamin, 1-(3-Aminophenyl)-3-methyl-5-pyrazolon, Pyrimidinamin, N-Aminomorpholin, 2-Aminobenzthiazol.

Besonders bevorzugte Amine sind Methylamin, Propylamin, Isopropylamin, n-Butylamin, sec.-Butylamin, tert.-Butylamin, Stearylamin, Hexamethylendiamin, Cyclohexylamin, 3,3,5-Trimethyl-5-aminomethyl-cyclohexylamin, 4,4'-Diaminodicyclohexylmethan, Anilin, p-Chloranilin, 3,4-Dichloranilin, m-Tolylamin, p-Methoxyanilin, 2,4-Diaminotoluol, 2,6-Diaminotoluol, 4,4'-Diaminodiphenylmethan, 2,4'-Diaminodiphenylmethan oder technische Gemische der genannten Diaminotoluole bzw. Diaminotoluole bzw. Diaminodiphenylmethane.

Weitere Ausgangsverbindungen für das erfindungsgemäße Verfahren sind

c) Alkohole der Formel

$$R_2{-}OH$$

in welcher

$R_2$ die bereits genannte Bedeutung hat.

Beispiele geeigneter Alkohole sind Methanol, Ethanol, Propanol, iso-Propanol, Butanol, iso-Butanol, Pentanol, iso-Pentanol, Hexanol, iso-Hexanol, Heptanol, iso-Heptanol, Octanol, iso-Octanol, Nonanol, iso-Nonanol, Decanol, iso-Decanol, Dodecanol, 2-Ethylhexanol, β-Chlorethanol, 2-Ethylbutanol, Hexadecanol, Octadecanol, Fettalkohol-Gemische, 2-Methoxyethanol, 2-Ethoxyethanol, 2-Propoxyethanol, 2-Butoxyethanol, 2-(2-Methoxyethoxy)-ethanol, 2-(2-Ethoxyethoxy)-ethanol, 2-(2-Butoxyethoxy)-ethanol, Cyclopentanol, Cyclohexanol, Methylcyclohexanol (und Gemisch), Cyclohexamethanol, 3,3,5-Trimethylcyclohexanol, 4-tert.-Butylcyclohexanol, 2-Hydroxydecalin, Borneol, Isoborneol, 1-(2-Hydroxyethoxy)-4-nitrobenzol, Benzylalkohol, 2-Phenylethanol, 2-(Methoxyphenoxy)-ethanol (Gemisch), 1-Phenylethanol, 3-Phenyl-1-propanol, 4-Methoxybenzylalkohol, Glykolmonomethyläther, Glykolmonoäthylether, Glykolmonopropyläther, Glykolmonoisopropyläther, Glykolmonobutyläther, Glykolmonoisobutyläther, Glykolmonophenyläther, Diglykolmonoethyläther, Diglykolmonomethylether.

Besonders bevorzugte Alkohole sind Methanol, Ethanol, n-Propanol, iso-Propanol, n-Butanol, iso-Butanol, Cyclohexanol, n-Hexanol, 2-Ethylhexanol, β-phenylethanol, Glykolmonomethylether, Glykolmonobutylether, Diglykolmonomethylether, Diglykolmonoethylether.

Bei der Durchführung des erfindungsgemäßen Verfahrens kommen die Amine b) im allgemeinen in 0,5-4-fach, vorzugsweise 0,8-1,5-fach und insbesondere 0,9-1,1-fach stöchiometrischen Mengen und die Alkohole c) im allgemeinen in 1-10-fach, vorzugsweise 1,1-4-fach und insbesondere 1,1-2-fach stöchiometrischen Mengen, jeweils bezogen auf die Carbonylgruppen der Komponente a), zum Einsatz.

Das erfindungsgemäße Verfahren wird in Gegenwart von Katalysatoren durchgeführt. Überraschenderweise wurde gefunden, daß beliebige Verbindungen, die einen katalytischen Einfluß auf die Veresterungsreaktion von Carbonsäuren mit Alkoholen ausüben auch eine beschleunigende Wirkung auf die erfindungsgemäße Umsetzung haben. Darüber hinaus bewirkt die Verwendung derartiger Verbindungen, daß in den Verfahrensprodukten, im Vergleich zur unkatalysierten Reaktion, ein weit geringerer Gehalt an N-unsubstituierten Urethanen der Formel

$$R_2{-}O{-}CO{-}NH_2$$

vorliegt. Für das erfindungsgemäße Verfahren besonders gut geeignete und bei der Herstellung von Di- und/oder Polyurethanen ausschließlich zu verwendende Katalysatoren sind (i) unter den Reaktionsbedingungen inerte anorganische oder organische Basin, (ii) Lewis-Säuren und (iii) Salze bzw. Komplexverbindungen, insbesondere Chelate von Übergangsmetallen.

Beispiele geeigneter Katalysatoren der Gruppe (i) sind tert.-Amine wie Tri-n-propylamin, Triethylamin, Triisopentylamin, Diethylbenzylamin, N,N-Dimethyl-benzylamin, Hexahydrodimethylanilin, N-Ethylpiperazin, Diethyl(2-methoxypropyl)-amin, 2-(Diethylaminoethyl)-phenylether, Oxethylmorpholin, N-(2-Diethylaminoethyl)-benzamid, N-(2-Diethylaminoethyl)-propionamid, 1,4-Diaza-(2,2,2)-bucyclooctan, N,N-Dimethyl-4-aminopyridin, 1-Azabicycloheptane, 1-Azabicyclooctane, gesättigte polyheterocyclische Amine, wie 3-Methylconidin, 1-Azabicyclo-(3,2,1)-octan, Pyrrolizidine und Chinuclidine, anorganische Basen wie basische Alkalisalze wie Natriumcarbonat, Natriumsulfid, Kaliumcarbonat oder Trinatriumphosphat

sowie Alkalisalze von Fettsäuren oder Sulfonsäuren.

Geeignete Katalysatoren (ii) sind beispielsweise Lewis-Säuren wie Eisen-II-chlorid, Eisen-III-chlorid, Zinkchlorid, Zinn-II-chlorid, Zinn-IV-chlorid, Aluminiumchlorid, Zinkcyanid, Thalliumtrichlorid, Bortrifluorid oder Bortrifluoridetherat.

Geeignete Katalysatoren der Gruppe (iii) sind beispielsweise Salze von Übergangsmetallen, soweit sie nicht bereits in die Gruppe (ii) fallen, sowie Komplexverbindungen insbesondere Chelate dieser Metalle wie Kobalt- oder Mangan-naphthenate, Eisenoleate oder -carbonyle, Acetylacetonate von Eisen, Nickel, Kobalt, Zink, Mangan, Molybdän, Titan, Thorium, Zirkon oder Vanadium, Bis-(dibenzoylmethan)-kupfer, Bis-(ethylacetoacetat)-kupfer, -eisen, Koordinationsverbindungen von Titan, Zirkon, Hafnium, Thorium und Mangan mit β-Diketonen, β-Ketoestern, und β-Hydroxyaldehyden, Acetate, Chloride, Sulfate oder Octoate des zwei- oder dreiwertigen Kobalts, des ein- oder zweiwertigen Kupfers oder des Zinks.

Weitere erfindungsgemäß geeignete Katalysatoren sind z. B. Bleinaphthenat, Acethylacetonate von Blei, Aluminium oder Magnesium, Dibutylzinndilaurat, Dibutylzinndiacetat, Di-(2-ethylhexyl)-zinnoxid, Dioctylzinnoxid, Zinnsalze von $C_1$-$C_{20}$-Carbonsäuren wie Zinn (II)-naphthenat, -hexoat- calmitat, -stearat oder -dimethyl- valerat, sowie Acetate, Chloride, Sulfate oder Octoate des zweiwertigen Bleis.

Besonders gut geeignete Katalysatoren sind beispielsweise Zinkchlorid, Zinkacetat, Zinkoctoat, Zinkoxid, Zinkcyanid, Zinn-II-chlorid, Zinn-IV-chlorid, Dibutylzinndilaurat, Kobalttriacetat, Kobaltrichlorid, Kobalttrioctoat, Kupfer-(II)-acetat, Kupfer-(I)-chlorid, Kupfer-(II)-sulfat, Bleiacetat oder Bleichlorid.

Die Menge des jeweils eingesetzten Katalysators liegt im allgemeinen zwischen 1 ppm und 20 Gew.-%, bevorzugt zwischen 100 ppm und 5 Gew.-%, bezogen auf die Summe der Ausgangsmaterialien a), b) und c). Man wird in der Praxis selbstverständlich bestrebt bleiben, die Konzentration der Katalysatoren möglichst gering zu halten. Die optimale Konzentration hängt von der Art der Ausgangsmaterialien und der Aktivität des jeweiligen Katalysators ab und kann in einem einfachen Vorversuch bestimmt werden.

Die Durchführung des erfindungsgemäßen Verfahren kann sowohl unter Druck als auch drucklos erfolgen. Die Anwendung von Druck beispielsweise im Bereich von 1 bis 80 bar ist dann oft sinnvoll, wenn die Reaktionstemperatur oberhalb dem Siedepunkt einer oder mehrerer der Ausgangsmaterialien liegt. Auch bei Verwendung von niedrigsiedenden Alkoholen ist man nicht unbedingt auf eine Durchführung der Umsetzung unter Druck angewiesen, da man auch in der Weise vorgehen kann, daß man die Reaktionspartner a) und b) auf die Reaktionstemperatur erhitzt und den niedrigsiedenden Alkohol so langsam zugibt, daß die Reaktionstemperatur erhalten bleibt. Dar erfindungsgemäße Verfahren wird im allgemeinen im Temperaturbereich von 120 °C bis 350 °C, vorzugsweise 130 bis 300 °C und insbesondere 140 °C bis 250 °C durchgeführt.

Das erfindungsgemäße Verfahren kann in Gegenwart oder auch in Abwesenheit von Lösungsmitteln durchgeführt werden. Geeignete Lösungsmittel sind beispielsweise unter den Verfahrensbedingungen inerte Lösungsmittel mit einem zwischen 100 °C und 280 °C, vorzugsweise zwischen 150 °C und 250 °C liegenden Siedepunkt. Beispiele geeigneter Lösungsmittel sind n-Nonan, n-Butylcyclohexan, Decahydronaphthalin, n-Undecan, n-Dodecan, n-Hexylcyclohexan, Dipenten, 1-Dodecen, Isopropylbenzol, 1,3-Diethylbenzol, Inden, n-Butylbenzol, Tetralin, Chlorbenzol, 4-Chlortoluol, 1,2-Dichlorbenzol, 2,4-Dichlortoluol, 1,2,4-Trichlorbenzol, 2-Chlor-4-isopropyl-1-methylbenzol, Anisol, Cyclohexylethylether, Diethylenglykoldimethylether, Benzylmethylether, 4-Methoxytoluol, Parachloranisol, Di-n-hexylether, Phenyl-n-propylketon, Benzophenon, Acetophenon, Formamid, N,N-Dimethylformamid, N,N-Diethylformamid, N-Methylformamid, Dimethylacetamid, N-Methylpyrrolidon, Caprolactam, Phenol, substituierte Phenole, Sulfolan, Hexamethylphosphorsäuretriamid, Dimethylsulfoxid, Ethylenglykolmonomethyletheracetat, Di-n-propylcarbonat, Cyclohexylacetat, Diisobutylcarbonat, Diethylenglykolmonomethyletheracetat, Diisoamylcarbonat, 2-Ethylpyridin, N,N-Dimethyl-2-methylanilin, N,N-Dimethylanilin, N-Methyl, N-Ethylanilin, N,N-Dimethyl-2-chloranilin, N,N-Diethylanilin, Chinolin, Nitrocyclohexan, Nitrobenzol, 2-Nitrotoluol, 2,4-Dimethyl-1-nitrobenzol, Acetonitril, N-Capronitril, Benzonitril, Tolunitril, Diphenyläther, Tetramethylharnstoff und Phenylacetonitril, besonders bevorzugt sind polare Lösungsmittel und deren Gemische. Zu den ganz besonders gut geeigneten Lösungsmitteln gehört ε-Caprolactam.

Die Mitverwendung derartiger Lösungsmittel ist oft, beispielsweise bei Verwendung eines hohen Überschusses an Alkohol (Komponente c)) überflüssig. Insbesondere erübrigt sich die Verwendung derartiger Hilfslösungsmittel bei der Herstellung Monourethanen (n = 1). d. h. bei der ausschließlichen Verwendung von Monoaminen als Komponente b).

Zur Durchführung des erfindungsgemäßen Verfahrens werden die Reaktionspartner im allgemeinen während eines Zeitraumes von 1 bis 15 Stunden auf die Reaktions-temperatur erhitzt, wobei darauf geachtet werden muß, daß der sich bildende Ammoniak entweichen kann. Die Aufarbeitung erfolgt in an sich bekannter Weise, insbesondere destillativ, wobei die Verfahrensprodukte meistens als letzte Fraktion oder als Destillationsrückstand anfallen. Vor dieser destillativen Aufarbeitung der Reaktionsansätze können erforderlichenfalls unlösliche Bestandteile (z. B. unlösliche Katalysatoren) abfiltriert werden. Die Verfahrensprodukte können dann ohne weitere Reindarstellung in an sich bekannter Weise thermisch in das ihnen zugrundeliegende Isocyanat und den ihnen zugrundeliegenden Alkohol gespalten werden.

Die in den nachfolgenden Beispielen erwähnten Prozentangaben beziehen sich auf Gewichtsprozente.

## Beispiel 1

Eine Füllkörperdruckkolonne aus Stahl (Nennweite 50 mm) mit einem Schlangenkühler als Kopfkondensator wird mit Füllringen aus Stahlmaschendrahtgewebe (4 mm) ca. 1 m hoch gefüllt. Als Sumpfgefäß der Kolonne wird ein Druckbehälter aus Stahl (Nenninhalt 5 l, zulässiger Höchstdruck 64 bar) angebracht, der mit einem Rührer und einer Mantelheizung versehen ist. Die Kolonne besitzt oberhalb des Kopfkondensators ein Ventil zur Entnahme von Gasen.

In den Druckbehälter werden 1 163 g 2-Ethyl-hexylamin, 541 g Harnstoff, 1 153 g Methanol und 5,0 g Zink-octoat eingefüllt. Nachdem der Druckbehälter und die Kolonne mit Stickstoff gespült wurden, wird das Gemisch unter Rühren erhitzt. Durch entsprechende Einstellung des Ventils am Kolonnenkopf sowie die Kühlleistung des Kopfkondensators wird der sich einstellende Druck in der Apparatur so reguliert, daß er gerade ausreicht, um die gewünschte Reaktionstemperatur zu ermöglichen. Das Gemisch wird auf 180 °C erhitzt und 4,5 Stunden auf 180 °C gehalten. Dabei wird freiwerdendes Ammoniak durch Rektifikation in der Kolonne von mitverdampfenden Substanzen getrennt und praktisch rein am Kolonnekopf gasförmig entnommen. Nach Beendigung der Reaktion wird das Gemisch abgekühlt, und die Apparatur wieder entspannt. Die Reaktionslösung wird gaschromatographisch analysiert. Nach Eichung mit einer authentischen Substanz wird mit Hilfe eines inneren Standards eine Ausbeute von 1 500 g (89 % der Theorie) N-(2-Ethyl-hexyl)-carbamidsäure-methylester ermittelt.

Beispiel 2

Entsprechend Beispiel 1 werden in der beschriebenen Druckapparatur 1 024 g Anilin, 671 g Harnstoff, 1 234 g Methanol und 6,2 g Zink-octoat unter Ammoniak-Entnahme 6,5 Stunden bei 200 °C umgesetzt. Nach dem Abkühlen und Entspannen der Apparatur wird das Reaktionsgemisch entnommen, filtriert und durch Destillation bei Normaldruck vom größten Teil des überschüssigen Methanols befreit. Anschließend wird das Gemisch bei 0,2 mbar fraktioniert destilliert, wobei als Hauptlauf 1 374 g (82,6 % der Theorie) N-Phenyl-carbamidsäure-methylester vom Schmelzpunkt 43-45 °C überdestillieren.

Beispiel 3

Entsprechend Beispiel 1 werden in der beschriebenen Druckapparatur 745 g Anilin, 480 g Harnstoff, 1 580 g Ethanol (ca. 96 %ig) und 5,9 g Zink-octoat unter Ammoniak-Entnahme 6,5 Stunden bei 200 °C umgesetzt. Nach dem Abkühlen und Entspannen der Apparatur wird das Reaktionsgemisch entnommen, filtriert und flüssigkeitschromatographisch (HPLC) untersucht. Nach Eichung mittels einer authentischen Substanz wird eine Ausbeute von 1 120 g (85 % der Theorie) N-Phenyl-carbamidsäure-ethylester ermittelt.

Beispiel 4

Entsprechend Beispiel 1 werden in der beschriebenen Apparatur 922 m-Toluidin, 517 g Harnstoff, 1 378 g Methanol und 5,8 g Zink-octoat Ammoniak-Entnahme 7,0 Stunden bei 190 °C umgesetzt. Nach dem Abkühlen und Entspannen der Apparatur wird das Reaktionsgemisch entnommen, filtriert und durch Flüssigkeitschromatographie (HPLC) analysiert. Nach Eichung mit einer authentischen Substanz wird eine Ausbeute von 1 210 g (85 % der Theorie) N-(3-Methyl-phenyl)-carbamidsäure-methylester ermittelt.

Beispiel 5

Entsprechend Beispiel 1 werden in der beschriebene Druckapparatur 810 g 3,4-Dichlor-anilin, 300 g Harnstoff, 927 g n-Butanol, 1 200 g o-Dichlorbenzol und 5,0 g Zinkoctoat unter Ammoniak-Entnahme 7,0 Stunden bei 200 °C umgesetzt. Nach dem Abkühlen und Entspannen der Apparatur wird das Reaktionsgemisch entnommen, filtriert und flüssigkeitschromatographisch (HPLC) untersucht. Nach Eichung mit einer authentischen Substanz wird eine Ausbeute von 1 140 g (87 % der Theorie) N-(3,4-Dichlor-phenyl)-carbamidsäure-n-butyl-ester ermittelt.

Beispiel 6

Entsprechend Beispiel 1 werden in der beschriebenen Druckapparatur 736 g trans, trans-4,4'-Diamino-dicyclohexyl-methan, 420 g Harnstoff, 1 683 g isopropanol und 5,7 g Zink-octoat unter Ammoniak-Entnahme 4,5 Stunden bei 190 °C umgesetzt. Nach dem Abkühlen und Entspannen der Apparatur wird das Reaktionsgemisch entnommen und durch Flüssigkeitschromatographie (HPLC) analysiert. Nach Eichung mit einer authentischen Substanz wird eine Ausbeute von 1 130 g (84 % der Theorie), 4,4'-Bis-(isopropoxycarbonyl-amino)-dicyclohexylmethan ermittelt.

Beispiel 7

Entsprechend Beispiel 1 werden in der beschriebenen Druckapparatur 648 g 2,4-Diamino-toluol, 637 g Harnstoff, 1 530 g Ethanol (ca. 96 %ig) und 5,3 g Zinkoctoat unter Ammoniak-Entnahme 6,0 Stunden bei

200 °C umgesetzt. Nach dem Abkühlen und Entspannen der Apparatur wird das Reaktionsgemisch entnommen, filtriert und flüssigkeitschromatographisch (HPLC) untersucht. Nach Eichung mit einer authentischen Substanz wird eine Ausbeute von 1 070 g (76 % der Theorie) 2,4-Bis-(ethoxycarbonylamino)-toluol ermittelt.

## Beispiel 8

In einem Rückflußkühler versehenen Reaktionsgefäß werden 1 Mol Anilin, 1 Mol Harnstoff und 1,4 Mol n-Hexanol in Gegenwart von 1 g Zinkchlorid zügig auf 140 °C erhitzt. Anschließend wird die Reaktionstemperatur allmählich innerhalb eines Zeitraums von 3 Stunden auf 200 °C erhöht. Nach weiteren 2 Stunden bei dieser Temperatur ist die Umsetzung beendet, was an der Beendigung der Ammoniakentwicklung erkannt wird. Die flüssigchromatographische Analyse des Reaktionsgemischs ergabe eine Ausbeute von 97 %, bezogen auf Harnstoff, an N-Phenyl-O-hexyl-urethan. Der Gehalt des Reaktionsgemischs an Carbaminsäurehexylester lag unter 1 %.

## Beispiel 9

744 g Anilin (8 Mol) werden mit 480 g Harnstoff (8 Mol), 15 ml Zinkoctoat und 1 200 g Cyclohexanol (12 Mol) gemischt und in den ersten 3 Ltr. Reaktor einer dreistufigen Kaskade gefüllt. Die drei Reaktoren werden auf 170 °C, 200 °C und 205 °C, erwärmt. Innerhalb von fünf Stunden wird ein Gemisch aus 1 860 g Anilin (20 Mol), 1 200 g Harnstoff (20 Mol), 25 ml Zinkoctoat und 3 kg Cyclohexanol (30 Mol), das zuvor auf 100 °C erwärmt wurde, in den ersten Reaktor gepumpt. Dann ist die Apparatur gefüllt und es werden erneut innerhalb von 5 Stunden ein Gemisch aus 1 660 g Anilin (20 Mol), 1 200 g Harnstoff (20 Mol), 25 ml Zinkoctoat und 3 kg Cyclohexanol (30 Mol) in die Kaskade gepumpt. Das fertige Reaktionsgemisch gelangt kontinuierlich in einen Dünnschichtverdampfer wo das Cyclohexanol im Wasserstrahlvakuum entfernt wird. Der Rückstand nach dieser Destillation besteht zu 96 % aus N-Phenyl-O-cyclohexylurethan (bestimmt durch Hochdruckflüssigkeitschromatographie = HPLC).

## Beispiel 10

170 g Isophorondiamin (1 Mol), 120 g Harnstoff (2 Mol) und 250 g Cyclohexanol werden mit 5 ml Zinkoctoat versetzt und das Reaktionsgemisch zügig auf 135 °C erwärmt, wobei die $NH_3$-Entwicklung einsetzt. Innerhalb von 6 Stunden wird die Innentemperatur auf 205 °C gesteigert und 3 Stunden bei dieser Temperatur gehalten. Nach dem Erkalten werden im Wasserstrahlvakuum 51 g Cyclohexanol abdestilliert. Es bleibt ein bei Raumtemperatur glasiges, durchsichtiges Harz zurück, das nach IR, KR fast vollständig aus dem gewünschtem Bisurethan besteht.

Ausbeute : 386 g 1-(Cyclohexoxycarbonyl-amino)-3,3,5-trimethyl-5-(cyclohexoxycarbonylamino-methyl)-cyclohexan (= 91 % d. Th.)

Fp. : 159-60 °C (aus Waschbenzin).

## Beispiel 11

122 g 2,4-Diaminotoluol (1 Mol), 120 g Harnstoff (2 Mol), und 250 g n-Hexanol (2,5 Mol) werden mit 1 ml Zinkoctoat versetzt und am Rückfluß erhitzt, bis die Sumpftemperatur 180 °C erreicht. Anschließend werden 160 ml n-Hexanol langsam zugetropft, sodaß die Reaktionstemperatur von 180 °C bei Rückflußbedingungen gehalten wird. Nach insgesamt 17 Stunden wird der Gehalt an gewünschtem Urethan hochdruckflüssigkeitschromatographisch zu 93 % d. Th. bestimmt. Das überschüssige Cyclohexanol wird im Vakuum abdestilliert und der Rückstand aus Isopropanol/Petroläther umkristallisiert. Es bleiben 308 g 2,4-Bis-(n-hexoxy-carbonyl-amino)-toluol (= 81 % d. Th.) vom Fp. 82-83 °C.

## Beispiel 11a

Es wird wie bei Beispiel 11 verfahren, jedoch 0,5 Mol 2,4-Diaminotoluol durch 0,5 Mol 2,6-Diaminotoluol ersetzt. Die Ausbeute an den gewünschten 2,4- und 2,6-Urethanen betrug 91 % d. Th.

## Beispiel 12

324 g 3,4-Dichloranilin (2 Mol), 120 g Harnstoff (2 Mol), 300 g Cyclohexanol (3 Mol) und 1 ml Zinkoctoat werden 13 Stunden am Rückfluß erhitzt, wobei die Reaktionstemperatur auf 205 °C ansteigt. Es bleiben ca. 655 g Rückstand in dem das gewünschte Urethan in einer Ausbeute von 90 % d. Th. vorliegt.

(Hochdruckflüssigkeitschromatographie).

Nach Umkristallisation aus Waschbenzin werden 470 g N-(3,4-Dichlorphenyl)-cyclohexylurethan (82 % d. Th.) vom Fp. 116-118 °C erhalten.

## Beispiel 13

183 g 3-Amino-dibenzofuran (1 Mol), 60 g Harnstoff (1 Mol), 150 g Cyclohexanol (1,5 Mol) und 2 ml Zinkacetat werden mit 200 g Caprolactam versetzt und zügig unter Ammoniakentwicklung auf 200 °C erwärmt. Nach 5 Stunden bei dieser Temperatur wird die Umsetzung beendet, das überschüssige Cyclohexanol abdestilliert.

Der Rückstand wird in Wasser gegeben, der unlösliche Anteil abfiltriert, getrocknet und aus waschbenzin umkristallisiert.

Ausbeute : 236 g N-(3-Dibenzofuranyl)-n-cyclohexyl-urethan (76 % d. Th.)

Smp. : 131-133 °C

## Biespiel 14

113 g 4-Morpholinamin (90 %ig, 1 Mol), 60 g Harnstoff (1 Mol), 150 g Cyclohexanol (1 Mol) und 2 g Zinkacetat werden zusammengegeben und auf 135 °C erwärmt, die Ammoniakentwicklung setzt ein. Die Temperatur wird langsam auf 190 °C gesteigert und dort 4 Stunden gehalten. Das überschüssige Cyclohexanol (52 g) wird im Wasserstrahlvakuum am Rotationsverdampfer entfernt, der Rückstand aus Waschbenzin umkristallisiert.

Ausbeute : 166 g N-(4-Morpholinyl-)-cyclohexylurethan (= 73 % d. Th.)

Fp. : 150-152 °C

## Beispiel 15

157 g 3-Aminosulfolan (86 %ig, 1 Mol), 60 g Harnstoff (1 Mol), 150 g Cyclohexanol (1,5 Mol) und 2 ml Zinkoctoat werden in einem 500 ml Dreihalskolben mit Rührer, Thermometer, und Rückflußkühler, gemischt und innerhalb von 4 Stunden auf 190 °C erwärmt, wobei der Ammoniak entweicht. Nach weiteren drei Stunden bei 193 °C wird die Umsetzung beendet, 45 g Cyclohexanol im Wasserstrahlvakuum entfernt und der Rückstand aus Essigester umkristallisiert.

Ausbeute : 241 g N-(3-Sulfolanyl)-cyclohexylurethan (≙ 92 % d. Th.)

Fp. : 139 °C

## Beispiel 16

324 g Benzylalkohol (3 Mol), 205 g Anilin (2,2 Mol) und 120 g Harnstoff (2 Mol) werden in einem 1 Ltr. Kolben gemischt, mit 1 g Zinkoctoat und 2 g Dibutylzinndilaurat versetzt und schnell auf 135 °C erwärmt wobei die $NH_3$-Entwicklung einsetzt. Innerhalb von drei Stunden wird die Innentemperatur auf 200 °C gesteigert und weitere 5 Stunden zwischen 195 °C und 205 °C gehalten.

Nach beendeter Umsetzung wird überschüssiger Benzylalkohol und Anilin im Wasserstrahlvakuum abdestilliert. Der Rückstand im Hochvakuum fraktioniert.

Ausbeute : 336 g N-Phenyl-benzyl-urethan (= 74 % d. Th.)

Sdp. : 142-145 °C bei 0,13 mbar

## Beispiel 17

94 g 2-Aminopyridin (1 Mol), 60 g Harnstoff (1 Mol), 150 g Cyclohexanol (1,5 Mol) werden mit 2 g Zinkcyanid versetzt und auf 135 °C erhitzt, wobei $NH_3$-Entwicklung einsetzt. Innerhalb von vier Stunden wird die Temperatur auf 200 °C gesteigert und dann weitere 2 Stunden bei dieser Temperatur belassen. Anschließend werden 55 g Cyclohexanol im Wasserstrahlvakuum abdestilliert, der Rückstand aus Waschbenzin umkristallisiert.

Ausbeute : 170 g N-(2-Pyridyl)-cyclohexylurethan (= 77 % d. Th.)

Fp. : 145-147 °C

## Beispiel 18

306 g n-Hexanol (3 Mol), 205 g Anilin (2,2 Mol) und 120 g Harnstoff (2 Mol) werden mit 2,5 ml Zinkoctoat versetzt und innerhalb von 5 Stunden, unter $NH_3$-Entwicklung, auf 200 °C erwärmt. Nach weiteren drei Stunden bei dieser Temperatur wird die Umsetzung beendet. Ueberschüssiges Cyclohexanol und Anilin werden im Wasserstrahlvakuum entfernt. Die Menge an gewünschtem Reaktionsprodukt wird durch HPLC bestimmt.

Ausbeute : 345 g N-Phenyl-n-hexylurethan (= 87 % d. Th.)

Fp. : 42-43 °C (aus Waschbenzin)

## Beispiel 19

127,5 g p-Chloranilin (1 Mol), 60 g Harnstoff (1 Mol) und 140 g Cyclohexanol (1,4 Mol) werden mit 3 g

Kobaltacetat versetzt und das Reaktionsgemisch auf 150 °C erwärmt. Es setzt eine Ammoniakentwicklung ein. Die Innentemperatur wird bis auf 200 °C gesteigert und dort 5 Stunden gehalten. Danach wird überschüssiges Cyclohexanol destillativ entfernt, der Rückstand aus Xylol umkristallisiert.

Ausbeute : 218 g N-(o-Chlorphenyl)-cyclohexylurethan (= 86 % d. Th.)

Fp. : 115-116 °C

Beispiel 20

116 g 1,6-Diaminohexan (1 Mol), 120 g Harnstoff (2 Mol), 250 g Cyclohexanol (2,5 Mol), 100 g Caprolactam und 3 ml Zinkoctoat werden auf 140 °C erwärmt, wobei die $NH_3$-Entwicklung einsetzt. Innerhalb von 4 Stunden wird die Innentemperatur auf 200 °C gesteigert und weitere 3 Stunden auf 205 °C erwärmt. Danach wird im Wasserstrahlvakuum Cyclohexanol und im Hochvakuum Caprolactam entfernt. Der Rückstand wird durch IR, KR, und HPLC identifiziert und bestimmt.

Ausbeute : 316 g 1,6-Bis-(cyclohexoxycarbonyl-amino)-hexan (≙ 86 % d. Th.)

Eine Probe wird aus Waschbenzin umkristallisiert.

Fp. : 110-111 °C

Beispiel 21

250 g Cyclohexanol (2,5 Mol), 120 g Harnstoff (2 Mol) und 3 ml Zinkoctoat werden auf 140 °C erwärmt und bei dieser Temperatur langsam 118 g Propylamin (2 Mol) zugetropft. Nach beendetem Zutropfen wird die Innentemperatur auf 200 °C gesteigert und eine Stunde bei dieser Temperatur nachgerührt. Das nicht umgesetzte Cyclohexanol wird im Wasserstrahlvakuum abdestilliert, der Rückstand an der Ölpumpe destilliert.

Ausbeute : 292 g N-(n-Propyl)-O-cyclohexyl-urethan (identifiziert durch HPLC mit einer authentischen Probe), (≙ 79 % d. Th.)

Beispiel 22

198 g 4,4'-Diamino-diphenylmethan (1 Mol), 120 g Harnstoff (2 Mol), 400 ml Cyclohexanol (4 Mol), 3 ml Zinkoctoat und 452 g Caprolactam werden gemischt und die Temperatur innerhalb von 3 Stunden auf 210 °C gesteigert. Nach 4 stündigem Erhitzen bei dieser Temperatur ist die $NH_3$-Abspaltung beendet. Das 40 °C wärme Reaktionsgemisch wird in Eiswasser eingerührt, mit Essigester aufgenommen und getrennt. Die Essigesterphase wird mehrmals mit Eiswasser gewaschen, dann über Natriumsulfat getrocknet und eingeengt. Der Rückstand wurde aus Xylol umkristallisiert.

Ausbeute : 415 g 4,4'-Bis-(cyclohexoxycarbonyl-amino)-diphenyl-methan (= 86 % d. Th.)

Fp. : 144 °C

Beispiel 23

258,3 g (2,1 Mol) 4-Methoxyanilin, 120 g (2 Mol) Harnstoff und 330,4 g (2,8 Mol) Glykolmonobutylether wurden mit 3 g Kobaltacetat 2 Stunden am Rückfluß erhitzt. Nachdem die Temperatur 200 °C erreicht hatte wurde weitere 8 Stunden bei dieser Temperatur gehalten. Nach Entfernen des Ueberschusses an Glykolmonobutylether wurde in 500 ml Toluol gelöst, vom unlöslichen Rückstand abgesaugt, mit 1 n HCl extrahiert und aufgearbeitet. Es blieb ein dünnschichtchromatographisch reines Öl, welches spektroskopisch als N-(4-Methoxyphenyl)-O-β-n-butoxyethylurethan identifiziert wurde. Die Ausbeute betrug 87 % d. Th.

Beispiel 24

204,6 g (2,2 Mol) Anilin, 120 g (2 Mol) Harnstoff und 354 g (3 Mol) Ethylenglykolmonobutylether wurden mit 0,8 g Dibutylzinndilaurat am Rückfluß erhitzt. Nach 2 Stunden waren 200 °C erreicht, die noch weitere 9 Stunden gehalten wurde. Die Lösung wurde eingeengt, der Rückstand mit heißem Ligroin behandelt ; vom unlöslichen Diphenylharnstoff wurde abgesaugt und das Ligroin wieder abdestilliert. Es blieb ein nicht kristallisierendes Öl, welches aus über 95 % N-Phenyl-O-β-n-butoxyethylurethan bestand, wie kernresonanzspektroskopisch nachgewiesen wurde. Die Aubeute betrug über 90 % d. Th.

Beispiel 25

204,6 g (2,2 Mol) Anilin, 120 g (2 Mol) Harnstoff und 366 g (3 Mol) β-Phenylethanol wurden mit 3 g Thalliumchlorid erhitzt. Die Rückflußtemperatur stieg während 1,5 Stunden auf 200 °C an. Bei nachlassender Ammoniakentwicklung wurde weitere 10 Stunden bei 200 °C gehalten. Mit Hilfe der Hochdruckflüssigkeitschromatographie wurde die Ausbeute an N-Phenyl-O-β-phenylethylurethan zu 90 % d. Th. bestimmt.

Beispiel 26

235,4 g (2,2 Mol) 3-Methylanilin, 120 g (2 Mol) Harnstoff und 390 g (3 Mol) 2-Ethyl-1-hexanol wurden mit 0,8 g Diazabicyclo [2.2.2.]-octan unter Ammoniakentwicklung 3 Stunden am Rückfluß erhitzt. Die Temperatur stieg dabei auf 200 °C an. Nach weiteren 7 Stunden bei 200 °C wurde das Reaktionsgemisch im Vakuum eingeengt, der Rückstand in 500 ml Waschbenzin heiß aufgenommen, und vom unlöslichen Anteil abgesaugt. Aus dem Filtrat wurde destillativ (0,2 mbar, 147-150 °C) O-2-Ethylhexyl-(1)-N-phenylurethan in 85 %iger Ausbeute isoliert.

Beispiel 27

354 g (1,2 Mol) Ethylenglykolmonobutylether, 120 g (2 Mol) Harnstoff und 186,4 g (1,2 Mol) 3-Aminotetramethylensulfon wurden mit 0,8 g Zinkoctoat am Rückfluß erhitzt. Nach 3 Stunden wurden 200 °C erreicht, die weitere 8 Stunden beibehalten wurden. Durch Destillation bei 0,6 mbar wurden die recyclisierbaren Ausgangs- und Nebenprodukte entfernt (Temperatur : bis 115 °C). Der Rückstand bestand nach infrarot- und kernresonanzspektroskopischer Untersuchung aus N-(3-Perhydrothiophenyl-1,1-dioxo)-O-β-n-butoxyethylurethan, welches in 85 %iger Ausbeute anfiel.

Beispiel 28

198 g (2 Mol) Cyclohexylamin, 132 g (2,2 Mol) Harnstoff und 244 g (2 Mol) β-Phenylethanol wurden mit 0,2 g Zinkoctoat am Rückfluß auf 200 °C erhitzt. Anschließend wurden weitere 122 g (1 Mol) β-Phenylethanol zugegeben und 4 Stunden bei 200 °C gehalten. Nach Aufarbeitung blieben 79 % d. Th. an N-Cyclohexyl-O-β-phenylethylurethan vom Schmelzpunkt 86-88 °C (Waschbenzin).

Beispiel 29

103 g (1 Mol) Biuret, 186 g (2 Mol) Anilin, 300 g (3 Mol) Cyclohexanol und 0,5 g Zinkoctoat wurden 4 Stunden am Rückfluß erhitzt, wobei die Temperatur 200 °C erreichte. Es wurde weitere 4 Stunden bei 200 °C gehalten. Die Ausbeute an O-Cyclohexyl-N-phenylurethan wurde hochdruckflüssigkeitschromatographisch zu 92 % d. Th. bestimmt.

Die Reaktion wurde ohne Katalysator wiederholt, jedoch wurde 20 Stunden bei 200 °C gehalten. Die Ausbeute an O-Cyclohexyl-N-phenylurethan wurde hochdruckflüssigkeitschromatographisch zu 60 % d. Th. bestimmt.

Beispiel 30

272 g (2 Mol) 4-Nitroanilin, 120 g (2 Mol) Harnstoff, 300 g (3 Mol) Cyclohexanol und 0,8 g Zinkoctoat wurden am Rückfluß erhitzt und nachdem 200 °C erreicht waren 7 Stunden dort gehalten. Durch Umkristallisation aus Ethanol/Wasser wurden 327 g = 62 % d. Th. an O-Cyclohexyl-N-4-nitrophenylurethan vom Schmelzpunkt 117 °C erhalten.

Nach obiger Vorschrift wurde 3-Nitroanilin umgesetzt. Es wurden 370 g = 70 % d. Th. an O-Cyclohexyl-N-3-nitrophenylurethan vom Schmelzpunkt 115 °C erhalten.

Nach obiger Vorschrift wurde 2-Nitroanilin umgesetzt. Es wurden 208 g = 43 % d. Th. an O-Cyclohexyl-N-2-nitrophenylurethan vom Schmelzpunkt 104 °C erhalten.

Beispiel 31

204,6 g (2,2 Mol) Anilin, 132 g (2,2 Mol) Harnstoff, 300 g (3 Mol) Cyclohexanol, 0,8 g Zinkoctoat und 0,2 g Zinnoctoat wurden unter Rückfluß erhitzt, wobei innerhalb von 2 Stunden die Reaktionstemperatur von 150 °C auf 200 °C anstieg. Bei 200 °C wurde weitere 8 Stunden gehalten. Während der Reaktion wurde kontinuierlich Ethanol (50 ml/Stunde) über die Lösung geleitet und über Kopf abgenommen. Die Urethanausbeuten wurden hochdruckflüssigkeitschromatographisch ermittelt. Es hatten sich 95,4 % d. Th. O-Cyclohexyl-N-phenylurethan und 3,7 % d. Th. O-Ethyl-N-phenylurethan gebildet.

Beispiel 32

204,6 g (2,2 Mol) Anilin, 120 g (2 Mol) Harnstoff, 300 g (3 Mol) Cyclohexanol und 0,6 g Zinkoctoat wurden am Rücksfluß erhitzt wobei innerhalb von 2 Stunden die Reaktionstemperatur von 150 °C auf 200 °C anstieg. Bei nachlassender Ammoniakentwicklung wurde weiter bei 200 °C gehalten, wobei der Reaktionsverlauf mit Hilfe der Hochdruckflüssigkeitschromatographie laufend verfolgt wurde. Nach 6 Stunden wurde die Ausbeute an O-Cyclohexyl-N-phenylurethan zu 97,9 % d. Th. bestimmt.

Nach Aufarbeitung wurden 416 g = 95 % d. Th. des Urethans vom Schmelzpunkt 80 °C erhalten.

Beispiel 33

Phenylcarbamidsäurebutylester

In einem 500 ml Dreihalskolben, der mit Rüher, Innenthermometer und Rückflußkühler ausgerüstet ist, werden 60,06 g (1 Mol) Harnstoff, 102,4 g (1,1 Mol) Anilin und 0,5 bzw. 2 g Katalysator auf 150 °C erhitzt. Man tropft unter Rühren und weiterem Erwärmen im Verlauf von 6 Std. 150-200 ml Butanol zu. Die Tropfrate wird so gewählt, daß sich eine Reaktionstemperatur von 175 °C einstellt. Der Umsatz kann über die Ammoniakentwicklung abgeschätzt werden.

Die Aufarbeitung erfolgt durch Destillation des Carbamats.

Ausbeute an N-Phenyl-O-butyl-urethan (HPLC des Reaktionsgemisches):

| | |
|---|---|
| unkatalysiert: | 140,6 g ≙ 72,8 % |
| Katalyse: 2 g $Cu(OAc)_2 \cdot H_2O$ | 161,0 g ≙ 83,4 % |
| 0,5 g Octasoligen-Zn | 179,2 g ≙ 92,9 % |

## Beispiel 34

186 g Anilin (2 Mol), 120 g Harnstoff (2 Mol), 300 ml Cyclohexanol (3 Mol) und jeweils 3 g Katalysator werden 9 Stunden am Rückfluß gekocht, wobei die Innentemperatur ansteigt. Nach dieser Zeit wird das überschüssige Cyclohexanol im Wasserstrahlvakuum entfernt und die Ausbeute an N-Phenyl-O-cyclohexylurethan durch HPLC bestimmt.

Die folgende Tabelle gibt einen Überblick über die Wirkung der verschiedenen Katalysatoren im Vergleich zu dem unkatalysierten Ansatz. die Zahlenangaben beziehen sich auf Molprozente.

| | |
|---|---|
| Zn-octoat | 97 |
| TlCl | 95 |
| $ZnCl_2$ | 95 |
| $ZnSO_4$ | 94,5 |
| $NiCl_2$ | 94,4 |
| Diazabicyclo [2.2.2]-octan | 94 |
| Co-naphtenat | 94 |
| 2,3,4,6,7,8,9,10-Octahydropyrimido [1,2-a] azepin | 92,5 |
| $TiCl_4$ | 92 |
| Zn-Staub | 92 |
| 2-Hydroxypyridin | 92 |
| K-Acetat | 92 |
| $BiCl_3$ | 92 |
| $BF_3$-Etherat | 91,5 |
| $K_2CO_3$ | 91 |
| Dibenzylamin | 91 |
| $AlCl_3$ | 91 |
| Zn-acetat | 91 |
| Fe-acetylacetonat | 90,5 |
| Cu-acetat | 90 |
| Na-methylat | 90 |
| $Zn(CN)_2$ | 90 |
| ZnO | 90 |
| Sn-octoat | 89,5 |
| ohne Katalysator | 81 |

## Beispiel 35

Ein 31-Vierhalskolben wird mit Rührer, Innenthermometer, Rückflußkühler, beheizbarer Ethanolzuleitung und Gasableitung ausgerüstet. Die Gasableitung führt zu einer Waschkolonne, die Ethanolförderung erfolgt über eine Telab-Pumpe. Man legt 814 g Harnstoff 1 302 g Anilin und 7 g Dibutylzinnoxid vor und erhitzt innerhalb einer Stunde unter Rühren auf 170 °C. Bei Temperaturen über 120 °C wird Ammoniak freigesetzt. Man pumpt nun überhitzten Ethanoldampf in das Reaktionsgemisch. Die Fördermenge beträgt anfangs 400 g/h und wird im Verlauf der Reaktion kontinuierlich reduziert, so daß die Reaktionstemperatur von 170 °C eingehalten werden kann. Nach 10 Stunden entstehen laut Flüssigkeitschromatographie 1 779 g (77 % d. Th.), nach 23 Stunden 1 917 g (83 % d. Th.) N-Phenylcarbamidsäureethylester. Die Destillation bei 110 °C/0,13 mbar ergibt 1 746 g (76 % d. Th.) N-Phenylcarbamidsäureethylester mit einem Gehalt von 1,8 Gew.-% N,N'-Diphenylharnstoff und 30 ppm Zinn. Der Destillationsrückstand beträgt 379 g ; er enthält 21 Gew.-% N-Phenylcarbamidsäureethylester, 61 Gew.-% N,N'-Diphenylharnstoff und 0,85 % Zinn.

Beispiel 36

Ein 0,5 l-Vierhalskolben wird mit Rührer, Tropftrichter und Rückflußkühler mit Gasableitung ausgerüstet. Das Einleitungsrohr des Tropftrichters taucht in das Reaktionsgemisch und wird mit Stickstoff gespült. Man legt im Kolben 60,1 g Harnstoff, 93,1 g Anilin und 0,5 g Dimethylzinndichlorid vor und erhitzt unter Rühren und Einleiten von Stickstoff auf 170 °C. Bei Temperaturen von über 120 °C wird Ammoniak freigesetzt. Man tropft nach Erreichen der Reaktionstemperatur 73 g Ethanol in dem Maße zu, daß die Reaktionstemperatur zwischen 170 und 175 °C gehalten werden kann. Nach 10 Stunden wird die Reaktion abgebrochen. Laut Flüssigkeitschromatographie sind 140,3 g (85 % d. Th.) N-Phenylcarbamidsäureethylester entstanden.

Beispiel 37

144 g (2,4 Mol) Harnstoff, 480 g (4 Mol) Diethylenglykolmonomethylether und 0,8 g Zinkoctoat werden 1 Stunde am Rückfluß erhitzt. Bei 200 °C werden 198 g (1 Mol) 4,4'-Diaminodiphenylmethan sowie soviel Diethylenglykoldimethylether langsam zugetropft, daß die Sumpftemperatur bei Rückfluß 200 °C nicht übersteigt. Nach 9 Stunden hatten sich 90 % d. Th. an 4,4'-Bis-[2-(2'-Methoxyethoxy)ethoxycarbonylamino] diphenylmethan gebildet (HPLC).

**Patentansprüche**

1. Verfahren zur Herstellung von Urethanen durch Umsetzung von a) Harnstoff oder Polyureten mit b) primären Aminen und c) Alkoholen im Temperaturbereich von 120° bis 350 °C in Gegenwart von Katalysatoren, wobei im Falle der Herstellung von Aryl-mono-, -di- und/oder -polyurethanen die zwingende Anwesenheit von O-Alkylcarbamidsäureestern im Ausgangsgemisch, und zwar in einer solchen Menge, daß das Verhältnis von Aminogruppen der primären aromatischen Amine zu O-Alkylcarbamidsäureestern bei 1 : 0,5 oder darunter liegt, ausgenommen ist, dadurch gekennzeichnet, daß man die Umsetzung in Gegenwart von beliebigen Veresterungskatalysatoren für Carbonsäuren durchführt mit der Einschränkung, daß im Falle der Herstellung von Di- und/oder Polyurethanen die Veresterungskatalysatoren aus der Gruppe bestehend aus (i) unter den Reaktionsbedingungen inerten anorganischen oder organischen Basen, (ii) Lewis-Säuren, (iii) Salzen bzw. Komplexverbindungen von Übergangsmetallen mit Ausnahme der Alkoholate des Titans, und (iv) Bleinaphthenat, Acetylacetonaten von Blei, Aluminium oder Magnesium, Dibutylzinndilaurat, Dibutylzinndiacetat, Di-(2-ethylhexyl)-zinnoxid, Dioctylzinnoxid, Zinnsalzen von $C_1$-$C_{20}$-Carbonsäuren, sowie Acetaten, Chloriden, Sulfaten oder Octoaten des zweiwertigen Bleis ausgewählt werden.

2. Verfahren gemäß Anspruch 1, dadurch gekennzeichnet, daß man die Umsetzung in Gegenwart von Übergangsmetallchelaten durchführt.

3. Verfahren gemäß Anspruch 1 und 2, dadurch gekennzeichnet, daß man die Umsetzung in Gegenwart von polaren Lösungsmitteln durchführt.

**Claims**

1. Process for the production of urethanes by reacting a) urea of polyurets with b) primary amines and c) alcohols in a temperature range of 120° to 350 °C in the presence of catalysts, with the proviso that, in the case of the production of aryl-mono-, -di- and/or polyurethanes, the imperative presence of O-alkylcarbamic acid esters in the starting mixture in such a quantity that the ratio of amino groups of the primary aromatic amines to O-alkylcarbamic acid esters is 1 : 0.5 or lower, is excluded, characterised in that the reaction is carried out in the presence of any desired esterification catalysts for carboxylic acids, with the proviso that, in the case of the production of di- and/or polyurethanes, the esterification catalysts are selected from the group consisting of (i) inorganic or organic bases which are inert under the reaction conditions, (ii) Lewis acids, (iii) salts or complex compounds of transition metals, with the exception of the alcoholates of titanium, and (iv) lead naphthenate, acetyl acetonates of lead, aluminium or magnesium, dibutyl tin dilaurate, dibutyl tin diacetate, di-(2-ethylhexyl) tin oxide, dioctyl tin oxide, tin salts of $C_1$-$C_{20}$-carboxylic acids, and acetates, chlorides, sulphates or octoates of divalent lead.

2. Process according to Claim 1, characterised in that the reaction is carried out in the presence of transition metal chelates.

3. Process according to Claim 1 and 2, characterised in that the reaction is carried out in the presence of polar solvents.

**Revendications**

1. Procédé pour la fabrication d'uréthannes par réaction a) d'urée ou de polyurets avec b) des

amides primaires et c) des alcools dans un intervalle de températures de 120 à 350 °C en présence de catalyseurs, la présence obligatoire dans le mélange de départ de carbamates de O-alkyles en quantité telle que le rapport des groupes amino des amines aromatiques primaires aux carbamates de O-alkyles soit de 1 : 0,5 ou au-dessous étant exclue dans le cas de la fabrication d'aryl-mono-, -di- et/ou -polyuréthannes, caractérisé en ce que l'on effectue la réaction en présence de catalyseurs quelconques d'estérification des acides carboxyliques, avec cette limite que, dans le cas de la fabrication de di- et/ou polyuréthannes, les catalyseurs d'estérification sont choisis dans le groupe constitué par (i) les bases inorganiques ou organiques inertes dans les conditions de réaction, (ii) les acides de Lewis, (iii) les sels ou composés complexes de métaux de transition, à l'exception des alcoolates du titane et (iv) le naphténate de plomb, les acétylacétonates de plomb, aluminium ou magnésium, le dilaurate de dibutylétain, le diacétate de dibutylétain, l'oxyde de di-(2-éthylhexyl)-étain, l'oxyde de dioctylétain, les sels d'étain d'acides carboxyliques en $C_1$-$C_{20}$, ainsi que les acétates, chlorures, sulfates ou octoates du plomb divalent.

2. Procédé selon la revendication 1, caractérisé en ce que la réaction est effectuée en présence de chélates de métaux de transition.

3. Procédé selon la revendication 1 ou 2, caractérisé en ce que l'on effectue la réaction en présence de solvants polaires.